# EUROPEAN PATENT APPLICATION

(11) **EP 4 437 838 A1**
(43) Date of publication of application: **02.10.2024**
(21) Application number: 24166286.5
(22) Date of filing: 26.03.2024
(51) Int. Cl.: A01H 1/00, A01H 5/04, A01H 6/64, C12N 9/02

(54) **MODIFIED PLANT**

(30) Priority: 28.03.2023 GB 202304476
(71) Applicant: Sun Pharmaceutical Industries Australia PTY Limited, Notting Hill VIC 3168 (AU)
(72) Inventor: GRAHAM, Ian Alexander, York (GB); MEADE, Fergus, Carlow (IE); WINZER, Thilo, York (GB); WALKER, Tracy Carol, Hindmarsh Island (AU); TOMAZ, Tiago, Gold Coast (AU)
(74) Representative: Symbiosis IP Limited

(57) **Abstract**

We discloses a plant of the genus *Papaver* that comprises one or more genomic modifications in multiple genes encoding one or more thebaine 6-O-demethylase genes (T6ODM) to reduce or knockout thebaine 6-O-demethylase activity wherein said plant is characterised by an increased oripavine content when compared to an unmodified plant of the same species.

## Description

### Field of the Disclosure

The disclosure relates to a plant of the genus *Papaver* that comprises one or more genomic modifications in multiple genes encoding one or more thebaine 6-O-demethylase genes (T6ODM) to reduce or knockout thebaine 6-O-demethylase activity wherein said plant is characterised by an increased oripavine content when compared to an unmodified plant of the same species.

### Background to the Disclosure

The biosynthetic pathway of morphinan alkaloids is well established and includes a series of enzymatic and non-enzymatic steps starting from dopamine and 4-hydroxyphenylacetaldehyde leading to the synthesis of R-reticuline which is subsequently transformed to thebaine. Thebaine is then converted to either oripavine by the codeine 3-O-demethylase (CODM) or transformed in several steps to codeine involving the activity of the thebaine-6-demethylase (T6ODM). Codeine is subsequently converted to morphine by de-methylation. Thebaine is the starting material for the synthesis of most semisynthetic opioids. Whilst opiate alkaloids can be extracted from latex, harvested from the green seed pods of opium poppy or from the poppy straw which is the dried mature plant, the demand for the opiate alkaloids in general exceeds the available natural supply necessitating costly chemical synthesis. Especially thebaine the chemical precursor of codeine, morphine and semisynthetic opioids is readily converted in the plant and thus the abundance is in general low. Oripavine is an opiate and the major metabolite of thebaine. It is the parent compound from which semisynthetic opioids are derived, which includes the compounds etorphine and buprenorphine.

The production of opiates is primarily through extraction from poppy, *Papaver somniferum.* There are examples of poppy plants that have been modified to introduce genomic mutations that result in altered opiate content, WO2017/122011 discloses modified poppy plants that are deleted for each of three copies of codeine demethylase (CODM). The modified plants do not express detectable levels of CODM expression or activity confirming that the genome of *Papaver somniferum* has only three copies of the CODM gene. In addition, the plants deleted for CODM genes advantageously have elevated levels of codeine. EP3398430 discloses poppy plants that are deleted for each of 5 copies of thebaine O- demethylase (T6ODM). In double mutants that carry deletions of each of the three CODM genes and each of the five T6ODM genes the modified plants have elevated levels of thebaine. WO2015/173590 discloses mutations in a cytochrome P450 oxidoreductase. The gene, Storr, encodes a fusion polypeptide comprising a cytochrome P450 domain and a oxidoreductase domain that catalyzes the conversion of (*S*) reticuline to (*R*)- reticuline. The complete genome sequence of *Papaver somniferum* is known and disclosed in Li Guo et al. The Opium Poppy Genome and Morphinan production: Science 362,343-347(2018).DOI:10.1126/science.aat4096 and can be found at NCBI ASM357369v1.

The disclosure relates to a modified poppy plant having elevated levels of oripavine when compared to an unmodified wild type plant. The modified poppy plant carries a mutation in a cluster of T6ODM genes wherein said mutation deletes each of five copies of the T6ODM genes to render the plant lacking detectable expression of T6ODM mRNA and protein. The modified poppy plants have elevated levels of oripavine when compared to an unmodified wild-type poppy.

### Statements of Invention

According to an aspect of the invention there is provided a *Papaver* plant wherein the plant is modified and comprises: a genomic deletion to all or part of five genes encoding thebaine 6-O-demethylases, wherein the expression or activity of said five thebaine 6-O-demethylases is undetectable and wherein the modified plant has an oripavine content that is at least 0.50 wt% of dried straw when compared to a wild type *Papaver* plant comprising functional genes encoding thebaine 6-O-demethylases.

"Modified" in the context of the invention includes the use of mutagenic agents that introduce mutations into genomic DNA thereby creating non-natural modifications to genes and/or genomic sequences. Mutations can be induced using chemical mutagens or physical mutagens, for example irradiation, that can introduce both point mutations or large deletions of genomic DNA thereby resulting in reduced expression or knockout of one or more genes. A commonly used chemical mutagen for introduction of mutations into plant genomic DNA is ethyl methane sulfonate (EMS). To introduce larger deletions of genomic DNA the preferred method is exposure of seed to a radiation source. Typically, radiation can be gamma radiation or neutron radiation by using the technique of Fast Neutron Mutagenesis (FNM). These techniques involve the exposure of plant seeds to a non-natural dosage of radiation, certainly higher than plants would be exposed to natural sources of radiation such as in sunlight.

The SI unit of ionising radiation dose is the Gray (Gy). Doses of ionising radiation used in mutagenesis of plant seed ranges from 1Gy to 500Gy. The ionising dose of radiation can vary depending on the plant species to which irradiation is applied.

In a preferred embodiment of the invention said irradiation dosage is 5-500 Gy.

In a preferred embodiment of the invention said irradiation dosage is 10-400 Gy.

In a preferred embodiment of the invention said irradiation dosage is 10-300 Gy.

In a preferred embodiment of the invention said irradiation dosage is 10-200 Gy.

In a preferred embodiment of the invention said irradiation dosage is 10-100 Gy.

Alternatively, said irradiation dosage is 400-500 Gy.

In a further preferred embodiment of the invention said irradiation dosage is 10-50 Gy or 50 to 60, 70, 80, 90 or 100 Gy.

In a preferred embodiment of the invention said mutated plant is not naturally occurring.

In a preferred embodiment of the invention said Papaver plant is *Papaver somniferum.*

In a preferred embodiment of the invention said genomic deletion comprises deletion of all or part of the nucleotide sequence set forth in SEQ ID NO: 1, wherein said nucleotide sequence comprises each of said five thebaine 6-O-demethylase genes or a polymorphic sequence variant thereof.

In a preferred embodiment of the invention said modified plant comprises an oripavine content of greater than 1.00 wt % of dried straw of said modified plant.

In a preferred embodiment of the invention said modified plant comprises an oripavine content of between 1.50 wt % and 2.00 wt% of dried straw of said modified plant.

In a preferred embodiment of the invention said modified plant comprises an oripavine content selected from the group: 1.10wt%, 1.15wt%, 1.20wt%, 1.25wt%, 1.30wt%, 1.35wt%, 1.45wt%, 1.50wt%, 1.55wt%, 1.60wt%, 1.65wt%, 1.70wt%, 1.75wt%, 1.80wt%, 1.85wt%, 1.90wt%, 1.95wt%, and 2.00 wt% of dried straw of said modified plant.

In a preferred embodiment of the invention said modified plant comprises an oripavine content of between 1.45 wt % and 1.55 wt% of dried straw of said modified plant.

In a preferred embodiment of the invention said modified plant comprises an oripavine content of about 1.50 wt % of dried straw of said modified plant.

In a preferred embodiment of the invention said modified plant comprises a thebaine content of at least 1.45 wt % of dried straw of said modified plant.

In a preferred embodiment of the invention said modified plant comprises a thebaine content of between 1.45 wt % and 2.60 wt % dried straw of said modified plant.

In a preferred embodiment of the invention said modified plant comprises a thebaine content selected from the group: 1.45wt% 1.50wt%, 1.55wt%, 1.60 wt%, 1.65 wt%, 1.70 wt%, 1.75 wt%, 1.80 wt%, 1.85 wt%, 1.90 wt%, 1.95 wt%, 2.00 wt% 2.50 wt%, 2.55 wt% and 2.60 wt%.

In a preferred embodiment of the invention said modified plant comprises a thebaine content of about 1.90 wt % dried straw of said modified plant.

In a preferred embodiment of the invention said modified plant comprises a oripavine content of about 1.00 wt% to 2.00 wt% and a thebaine content of 1.00 wt % and 2.60 wt % dried straw of said modified plant.

According to an aspect of the invention there is provided a seed or seed pod obtained or obtainable from a modified plant according to the invention.

According to a further aspect of the invention there is provided latex or dried straw obtained or obtainable from the modified plant according to the invention.

According to an aspect of the invention there is provided a process for the extraction of oripavine comprising the steps:
i) harvesting a plant or plant material prepared from a plant according to the invention;
ii) forming a reaction mixture of particulate plant material;
iii) solvent extraction of the reaction mixture to provide an alkaloid enriched fraction; and
iv) concentrating said alkaloid enriched fraction to provide an oripavine enriched fraction.

In a preferred method of the invention said plant material comprises poppy straw.

In an alternative method of the invention said plant material comprises poppy poppy latex.

"Total alkaloid content" is defined relative to the major alkaloids: morphine, codeine, noscapine, oripavine and thebaine. Dried straw refers to the dried stalks, stem and leaves of poppies minus the seeds.

Throughout the description and claims of this specification, the words "comprise" and "contain" and variations of the words, for example "comprising" and "comprises", means "including but not limited to", and is not intended to (and does not) exclude other moieties, additives, components, integers or steps. "Consisting essentially" means having the essential integers but including integers which do not materially affect the function of the essential integers.

Throughout the description and claims of this specification, the singular encompasses the plural unless the context otherwise requires. Where the indefinite article is used, the specification is to be understood as contemplating plurality as well as singularity, unless the context requires otherwise.

Features, integers, characteristics, compounds, chemical moieties, or groups described in conjunction with a particular aspect, embodiment or example of the invention are to be understood to be applicable to any other aspect, embodiment or example described herein unless incompatible therewith.

An embodiment of the invention will now be described by example only and with reference to the following figure:

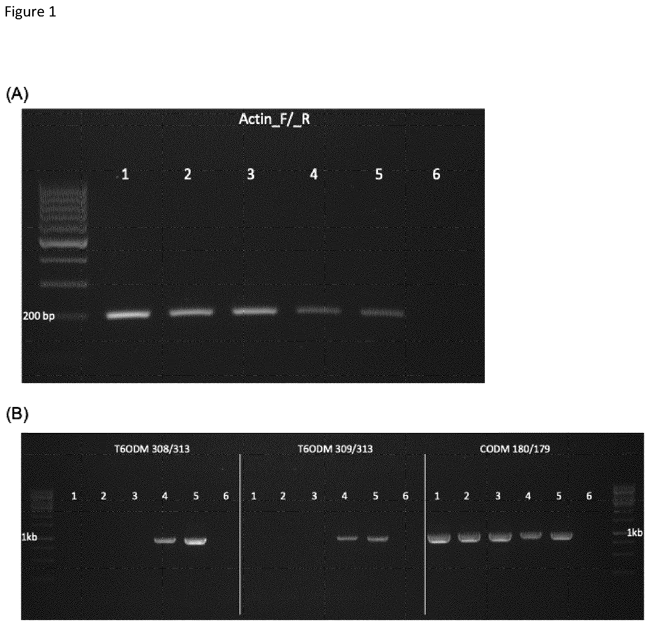

Figure 1: Amplification of *T6ODM, CODM* and *Actin* fragments from high oripavine plants from cultivar Y6 and control material. Lane 1, high oripavine Y6 plant Sd-773969; Lane 2, high oripavine Y6 plant Sd-773968; Lane 3, high oripavine Y6 plant Sd-773966; Lane 4, HN4 plant; Lane 5, HM8 plant; Lane 6, No Template Control; size marker: DNA size markers indicate size of amplified bands in base pairs as shown. The primer pair combinations used for fragment amplification are indicated and shown in Table 2 alongside the respective expected fragment lengths. (A) Amplification of *Actin* fragment resolved on 1.5% agarose gel. (B) Amplification of *T6ODM* and CODM fragments resolved on 1% agarose gel; and

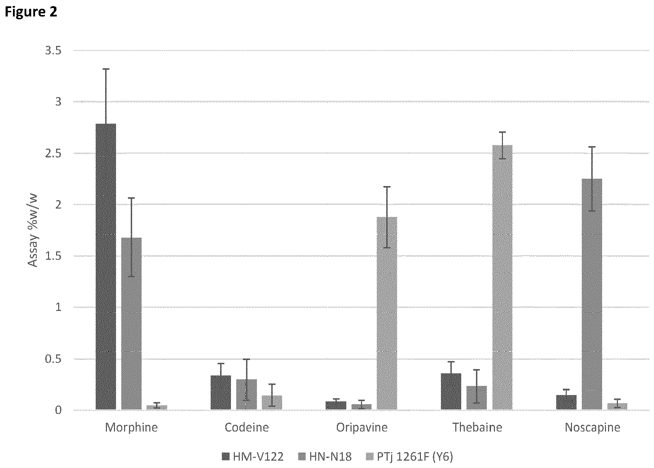

Figure 2: Quantification of concentration of the 5 major alkaloids from high oripavine cultivar Y6 and commercial check varieties HM-V122 and HN-N18. The average alkaloid quantities of morphine, codeine, oripavine, thebaine and codeine expressed on a % dry weight basis (±S.D) from capsule material harvested in replicated plots (n=4) across four cultivar trials implemented in the central, eastern and north western cultivation districts for High Morphine V122 (HM-V122), High Noscapine (HM-N18) and High Oripavine (PTj 1261F; Y6) cultivars.

### MATERIALS AND METHODS

### Poppy straw analysis of field-based material from Cultivar Trials

Cultivar trials were sown and managed within Sun Pharma leased paddocks situated in the Northern, North-Western, North-Eastern, Central and Midlands (northern and southern) regions of Tasmania. Paddocks were selected based on their expected plant growth regulator (PGR) spay applications with trials situated in crops designated with and without application of the PGR program. At each of the sites a visually homogenous region was selected; avoiding wheel marks, headlands and other paddock anomalies where possible. In the weeks prior to sowing, commercially relevant sowing densities were determined using a Climatron^{®} (Thermoline Scientific, Wetherill Park, Australia) temperature and humidity-controlled growth cabinet to determine percentage seed viability and a count of seeds per gram to maintain a seeding density of 1,500,000 viable seeds/Ha. Using a purpose build seed drill the cultivar trials were sown in a randomized complete block design comprising four replicated yield plots (8.775m2) of which a 2 x 2 meter quadrats was sampled. Trials were sown within a ± 12hr window following the cultivation of the soil and a pre-application of NPK applied by commercial drill contractors. Cultivars sown as commercial controls were separated into six major groupings; current commercial morphine (V), codeine (X), thebaine + oripavine (T), oripavine + thebaine (Y), noscapine + morphine (N), noscapine + thebaine (Z) and white seed varieties. These varieties were compared against varieties (YO) generated through the University of York marker assisted breeding program and advanced generation single or mixed alkaloid morphine (PMg), thebaine (PTg), noscapine (PNg) and white seed varieties developed through the hybridisation and the generational selfing program implemented in Tasmania. Cultivar trials were subject to commercial paddock management regimes, involving the application of various herbicide, fungicide and plant growth regulator applications, all to ensure optimal weed control and health of the commercial poppy crop throughout the season. A tinytag^{®} logger was situated at each of the cultivar sites recording temperature and humidity with additional information such as rainfall and historical weather observations being collected from the BOM (Commonwealth of Australia 2023, Bureau of Meteorology).

Poppy capsules were harvested from the respective cultivar lines by hand once capsules had dried to approximately 10% moisture on the plant. The seed was separated from the capsule, and capsule straw material (poppy straw) was either milled to a fine powder using a cutting mill (Model Pulverisette, Fritsch, Idar-Oberstein, Germany) and then shipped to Sun Pharma's extraction facility in Port Fairy, Australia; or shipped whole and milled to a finer consistency at the company's Victorian premises through use of a custom 900W NutriBullet blender (NutriBullet, Los Angeles, CA, USA). Two gram samples of ground poppy straw (2 ± 0.003g) were extracted in 50mL of a 10% acetic acid solution. The extraction suspension was shaken on an orbital shaker at 200rpm for a minimum of 10 minutes then filtered to provide a clear filtrate. The final filtrate was passed through a 0.22 µm filter prior to analysis.

The solutions were analysed using a Waters Acquity UPLC System (Waters Corporation, Milford, MA, USA) fitted with a Waters Acquity BEH C18 column, 2.1mm x 100mm with 1.7 micron packing. The mobile phase used a gradient profile with eluent A consisting of 0.1% Trifluoroacetic acid in deionised water and eluent B consisting of 100% Acetonitrile. The mobile phase gradient conditions used are as listed in Table 1, the gradient curve number as determined using a Waters Empower chromatography software package. The flow rate was 0.6 mL per minute and the column maintained at 45 degrees centigrade. The injection volume was 1 µL injection volume and the alkaloids were detected using a UV detector at 285nm.

The loss on drying (LOD) of the straw was determined by drying in an oven at 105 degrees centigrade for 16-20 hours. Alkaloid concentrations for morphine, pseudomorphine, codeine, thebaine, Oripavine and noscapine were quantified using external standard calibration and the results calculated on a dry weight basis. Reticuline was quantified using the thebaine calibration (assuming the thebaine response) and results calculated on a dry weight basis (% weight relative to thebaine; % WRT).

**Table1: Gradient Flow Program**

| **TIME (minutes)** | **% Eluent A** | **% Eluent B** | **Flow (mL/min)** | **Curve No** |
|---|---|---|---|---|
| 0.0 | 95 | 5 | 0.60 | INITIAL |
| 0.80 | 90 | 10 | 0.60 | 6 |
| 3.40 | 75 | 25 | 0.60 | 3 |
| 3.60 | 95 | 5 | 0.60 | 6 |
| 4.00 | 95 | 5 | 0.60 | 11 |

Typical retention times are as follows:

| **Compound** | **Retention Time (minutes)** |
|---|---|
| Morphine | 1.14 |
| Pseudomorphine | 1.26 |
| Codeine | 1.69 |
| Oripavine | 1.80 |
| Reticuline | 2.05 |
| 10-Hydroxythebaine | 2.32 |
| Thebaine | 2.53 |
| Noscapine | 3.16 |

### Plant Material

Plants for DNA extractions were grown under glass in 16-hour days at the University of York horticulture facilities. The growth substrate consisted of 4 parts John Innes No. 2, 1 part Perlite and 2 parts Vermiculite.

### Preparation of genomic DNA

For genomic DNA extraction 30-50 mgs of leaf material was collected from 4-6 week old glasshouse-grown plants. Genomic DNA was extracted using the BioSprint 96 Plant kit on the BioSprint 96 Workstation (Qiagen, Crawley, UK) according to the manufacturer's protocol.

Extracted DNA was quantified by spectrometry using the NanoDrop^{®} (Thermo Scientific, UK) and normalized to 5 or 10 ng/ml.

### Amplification of T6ODM and CODM fragments from genomic DNA

PCR amplification of *T6ODM* and CODM fragments was carried out on genomic DNA extracted from three individual plants (Sd-773966, Sd-773968 and Sd-773969) of Sun Pharmaceutical Industries (Australia) high oripavine cultivar Y6 and, as a control, from one individual plant each from morphine cultivar (HM8) and a noscapine cultivar (HN4). In addition, an actin fragment (ca. 200 bp in length) was amplified from all samples as a positive control to show that the extracted DNA was suitable for PCR amplification.

PCR reactions containing 5x GoTaq Flexi buffer, 0.2mM dNTPs, 1.0 µM forward and reverse primer (Table 1 and 2), 1U GoTaq Flexi DNA polymerase (Promega, M8305), 1.5 mM MgCl₂ and 10 ng of template DNA in a total volume of 10µl were run on a T100^{™} Thermal Cycler (Bio-Rad) using the following conditions:
a) Amplification of *Actin* fragment: Initial denaturation at 94°C for 2 minutes, followed by 35 cycles of 94°C for 30 seconds, 60°C for 30 seconds, 72°C for 20 seconds, followed by a final extension at 72°C for 5 minutes.
b) Amplification of *T6ODM* and *CODM* fragments: Initial denaturation at 94°C for 2 minutes, followed by 10 cycles of 94°C for 30 seconds, 65°C (decreasing to 60°C by 0.5°C per cycle) for 30 seconds, 72°C for 1 minute, followed by 25 cycles of 94°C for 30 seconds, 60°C for 30 seconds, 72°C for 1 minute, followed by a final extension of 72°C for 5 minutes.

PCR products were analysed by gel electrophoresis on 1-1.5% agarose gels. Primers and primer combinations used for amplification are shown in Tables 1 and 2.

**Table 1: Primers used for PCR amplifications**

| **Target gene** | **Primer_id** | **Orientation** | **sequence (5'->3')** |
|---|---|---|---|
| CODM | 179 | Reverse (SEQ ID NO 2) | CTCGACGCTACGGTAAATCC |
| | 180 | Forward (SEQ ID NO 3) | GTTAACCATGGAGTCGACGC |
| T6ODM | 308 | Forward (SEQ ID NO 4) | ACACCTAAGTCTCCCTATTTCTGC |
| | 309 | Forward (SEQ ID NO 5) | ACACCTAAGTCTCCCTATTTCTAT |
| | 313 | Reverse (SEQ ID NO 6) | ATAGTTGAATCTGACATGCAATCAC |
| Actin | Actin_F | Forward (SEQ ID NO 7) | GGGTACTCATTCACCACCACT |
| | Actin_R | Reverse (SEQ ID NO 8) | GGTTGGAAAAGCACCTCTGG |

**Table 2: Primer combination used for PCR amplifications shown in Figure 1**

| Fragment | Forward primer ID | Reverse primer ID | Expected fragment size [bp] |
|---|---|---|---|
| T6ODM | 308 | 313 | 946 (*T6ODM* genes 1 and 2); |
| | | | 945 *(T6ODM* gene 3) |
| T6ODM | 309 | 313 | 923 (*T6ODM* gene 4); |
| | | | 945 (*T6ODM* gene 5) |
| CODM | 180 | 179 | 1016 |
| Actin | Actin_F | Actin_R | ca 200 |

### Example 1

### Functional T6ODM genes are deleted in the high oripavine material

The opium poppy genome contains five full-length functional and locally linked *T6ODM* gene copies (Guo et al.,Science 362, 343-347 (2018)).

Using primers specific to the functional *T6ODM* gene copies 1 to 3 and to copies 4 and 5, respectively, failed to amplify fragments of any of the five functional and annotated *T6ODM* gene copies from DNA obtained from plants displaying the high oripavine phenotype (Figure 1). The amplification of both *Actin* and CODM fragments from the same DNA samples shows that the DNA was intact and suitable for amplification of *T6ODM* fragments of the expected size. Amplification of *T6ODM* fragments from positive control samples of morphine cultivar HM8 and noscapine cultivar HN4 demonstrates that the lack of amplification in plants from the high oripavine cultivar Y6 was not caused by *T6ODM* primer being unable to bind T6ODM gene sequence when present in genomic DNA. Taken together, the results from the PCR amplification clearly demonstrate that all functional *T6ODM* genes are deleted in high oripavine Y6 cultivar whereas CODM genes are present. These results are consistent with the high oripavine phenotype with the lack of T6ODM blocking metabolite flux from thebaine to codeine and the presence of CODM facilitating the conversion of thebaine to oripavine.

### Example 2

### Under field cultivation conditions, oripavine content is elevated in high oripavine material when compared to material containing functional T6ODM genes

Cultivar trials containing high morphine (HM-V122), high noscapine (HM-N18) and the high oripavine PTj 1261F (Y6) variety were implemented at Central, Eastern and North Western district locations (n=4) in the 2021/22 poppy cultivation season with standard sowing densities and agrochemical treatment programs (inclusive of PGR) utilised. Resultant capsule material harvested from these trials was harvested, extracted and assayed by UPLC and the quantities of the five major alkaloids (morphine, codeine, oripavine, thebaine and noscapine) were determined on a dry weight basis (Figure 2). The high oripavine Y6 cultivar displays on average approximately 23x the oripavine quantity of a high morphine cultivar and 34x the oripavine quantity of a high noscapine cultivar and contains a low morphinan background consistent with the absence of T6ODM in this cultivar blocking the conversion of Thebaine to Codeine and Oripavine to Morphine, and presence of CODM facilitating the conversion of Thebaine to Oripavine.

## Claims

1. A *Papaver* plant wherein the plant is modified and comprises: a genomic deletion to all or part of five genes encoding thebaine 6-O-demethylases, wherein the expression or activity of said five thebaine 6-O-demethylases is undetectable and wherein the modified plant has an oripavine content that is at least 0.50 wt% of dried straw when compared to a wild type *Papaver* plant comprising functional genes encoding thebaine 6-O-demethylases.

2. The *Papaver* plant according to claim 1 wherein said Papaver plant is *Papaver somniferum.*

3. The *Papaver* plant according to claim 1 or 2 wherein said genomic deletion comprises deletion of all or part of the nucleotide sequence set forth in SEQ ID NO: 1, wherein said nucleotide sequence comprises each of said five thebaine 6-O-demethylase genes or a polymorphic sequence variant thereof.

4. The *Papaver* plant according to any one of claims 1 to 3 wherein said modified plant comprises an oripavine content of greater than 1.00 wt % of dried straw of said modified plant.

5. The *Papaver* plant according to claim 4 wherein said modified plant comprises an oripavine content of between 1.50 wt % and 2.0 wt% of dried straw of said modified plant.

6. The *Papaver* plant according to claim 5 wherein said modified plant comprises an oripavine content selected from the group: 1.10wt%, 1.15wt%, 1.20wt%, 1.25wt%, 1.30wt%, 1.35wt%, 1.45wt%, 1.50wt%, 1.55wt%, 1.60wt%, 1.65wt%, 1.70wt%, 1.75wt%, 1.80wt%, 1.85wt%, 1.90wt%, 1.95wt%, and 2.00 wt% of dried straw of said modified plant.

7. The *Papaver* plant according to claim 6 wherein said modified plant comprises an oripavine content of between 1.45 wt % and 1.55 wt% of dried straw of said modified plant.

8. The *Papaver* plant according to claim 7 wherein said modified plant comprises an oripavine content of about 1.50 wt % of dried straw of said modified plant.

9. The *Papaver* plant according to any one of claims 1 to 8 wherein said modified plant comprises a thebaine content of at least 1.45 wt % of dried straw of said modified plant.

10. The *Papaver* plant according to claim 9 wherein said modified plant comprises a thebaine content of between 1.45 wt % and 2.60 wt % dried straw of said modified plant.

11. The *Papaver* plant according to claim 10 wherein said modified plant comprises a thebaine content selected from the group: 1.45wt% 1.50wt%, 1.55wt%, 1.60 wt%, 1.65 wt%, 1.70 wt%, 1.75 wt%, 1.80 wt%, 1.85 wt%, 1.90 wt%, 1.95 wt%,2.00 wt%, 2.50 wt%, 2.55 wt% and 2.60wt%.

12. The *Papaver* plant according to claim 11 wherein said modified plant comprises a thebaine content of about 1.90 wt % dried straw of said modified plant.

13. The *Papaver* plant according to any one of claims 1 to 12 wherein said modified plant comprises a oripavine content of about 1.00 wt% to 2.00 wt% and a thebaine content of 1.00 wt % and 2.60 wt % dried straw of said modified plant.

14. A seed or seed pod obtained or obtainable from a modified plant according to any one of claims 1 to 13.

15. Latex or dried straw obtained or obtainable from the modified plant according to any one of claims 1 to 13.

16. A process for the extraction of oripavine comprising the steps:
i) harvesting a plant or plant material prepared from a plant according to any one of claims 1 to 8;
ii) forming a reaction mixture of particulate plant material;
iii) solvent extraction of the reaction mixture to provide an alkaloid enriched fraction; and
iv) concentrating said alkaloid enriched fraction to provide an oripavine enriched fraction.

17. The process according to claim 16 wherein said plant material comprises poppy straw or poppy latex.
